(19) Europäisches Patentamt
European Patent Office
Office européen des brevets

(11) **EP 3 886 109 A1**

(12) **EUROPEAN PATENT APPLICATION**

(43) Date of publication:
**29.09.2021 Bulletin 2021/39**

(51) Int Cl.:
***G16H 20/30*** (2018.01)       ***A63B 71/06*** (2006.01)

(21) Application number: **20185591.3**

(22) Date of filing: **14.07.2020**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN** | (72) Inventor: **CHUANG, Lung-Fei**<br>**42083 Taichung City (TW)**<br><br>(74) Representative: **dompatent von Kreisler Selting Werner -**<br>**Partnerschaft von Patent- und Rechtsanwälten mbB**<br>**Deichmannhaus am Dom**<br>**Bahnhofsvorplatz 1**<br>**50667 Köln (DE)** |
| (30) Priority: **27.03.2020 TW 109110644** | |
| (71) Applicant: **Chuang, Lung-Fei**<br>**Taichung City 42083 (TW)** | |

(54) **SCORING METHOD AND SYSTEM FOR EXERCISE COURSE**

(57)     A scoring method for exercise course is provided and includes: playing a course video, obtaining coach exercise data corresponding to the course video, and obtaining a coach window from the coach exercise data; obtaining user exercise data through an inertial measurement unit, and obtaining a user window from the user exercise data in which the length of the user window is longer than that of the coach window; finding a user segment of the user window that is most similar to the coach window; calculating an exercise score according to stabilities of the user segment and the coach window for a first exercise type; and calculating the exercise score based on the difference between the user segment and the coach window by a deduction mechanism for a second exercise type.

FIG. 1A

**Description**

**BACKGROUND**

Field of Invention

The present disclosure relates to an exercise scoring method in which a score is calculated based on an exercise type.

Description of Related Art

With the rise of fitness in recent years, more and more people are willing to establish their own exercise habits. In today's information-rich environment, many people search for fitness-related instructional videos through resources such as the Internet and imitate the movements in the videos to exercise. However, since a person generally does not have the ability to perform the required action correctly by imitation, he or she may be injured by doing the wrong action before the exercise effect is achieved. In addition, in the absence of a couch to assist in the assessment, the person does not know whether he has gradually improved during the exercise, so he may not be willing to continue exercising due to lack of accomplishment.

A common motion assessing system uses a multi-lens photographing device with computer vision technology to analyze the user's motion posture, but this system costs a lot with respect to hardware or the production of digital content, causing difficulty in promotion. A conventional way is to use a wearable inertial sensor which includes an accelerometer and a gyroscope to record acceleration and angular velocity of the sensor in three-dimensional space. Thus, the motion trajectory can be calculated, analyzed, and compared. However, different exercise types have different characteristics, so how to properly analyze different exercise types is an issue for those skilled in the art.

**SUMMARY**

Embodiments of the present disclosure provide a scoring method for a system including a wearable device which includes an inertial measurement unit. The scoring method includes: playing a course video, obtaining coach exercise data corresponding to the course video, and obtaining a coach window from the coach exercise data; obtaining user exercise data from the inertial measurement unit, and obtaining a user window from the user exercise data in which a length of the user window is longer than that of the coach window; searching a user segment from the user window that is most similar to the coach window; for a first exercise type, calculating an exercise score according to stabilities of the user segment and the coach window; and for a second exercise type, calculating the exercise score based on a difference between the user segment and the coach window by a deduction mechanism.

In some embodiments, a beginning point of the user window precedes a beginning point of the coach window, and an end point of the user window follows an end point of the coach window. The step of searching the user segment from the user window that is most similar to the coach window includes: performing an open-begin-end dynamic time warping algorithm to the user window and the coach window to obtain the user segment.

In some embodiments, the step of calculating the exercise score according to the stabilities of the user segment and the coach window includes: calculating a mean absolute deviation of forces at sample points of the user segment as a user stability; calculating a mean absolute deviation of forces at sample points of the coach window as a coach stability; and calculating a stability ratio between the user stability and the coach stability.

In some embodiments, the step of calculating the exercise score according to the stabilities of the user segment and the coach window further includes: transforming the stability ratio into a stability score based on a logit function; calculating the corresponding coach stability for each window of the coach exercise data, counting times of the coach stabilities falling within a first range as a first number of times, and counting times of the coach stabilities falling within a second range as a second number of times; dividing the first number of times by a sum of the first number of times and the second number of times to obtain a stability weight; and calculating the exercise score according to the stability score and the stability weight.

In some embodiments, the step of calculating the exercise score according to the stability score and the stability weight is based on an equation (1).

$$Score = S_{stability} \times w + (1 - w) \times S_1 \qquad (1)$$

Score is the exercise score, $S_{stability}$ is the stability score, w is the stability weight, and $S_1$ is a real number.

In some embodiments, the step of calculating the exercise score based on the difference between the user segment and the coach window by a deduction mechanism includes: calculating a force difference and an angular difference between the user segment and the coach window; setting a force weight and an angular weight, and when larger one of the force difference and the angular difference is greater than an error threshold, increasing one of the force weight and the angular weight that corresponds to the larger one of the force difference and the angular difference; and calculating the exercise score based on the force difference, the angular difference, the force weight, and the angular weight.

In some embodiments, the force difference is based on differences between L2 norms of the user segment and L2 norms of the coach window at matched sample points. The angular difference is based on cosine similarities between the user segment and the coach window at the matched sample points.

In some embodiments, the step of calculating the exercise score based on the force difference, the angular difference, the force weight, and the angular weight is based on the following equation (2).

$$Score = 100 - (D_a \times w_a + D_m \times w_m) \qquad (2)$$

*Score* is the exercise score, $D_a$ is the angular difference, $W_a$ is the angular weight, $D_m$ is the force difference, and $W_m$ is the force weight.

From another aspect, embodiments of the present disclosure provide a system including a wearable device and a smart device. The wearable device includes an inertial measurement unit configured to obtain user exercise data. The smart device is communicatively connected to the wearable device for receiving the user exercise data and configured to play a course video corresponding to the course video through a display. The smart device is configured to perform multiple steps or transmit the user exercise data and coach exercise data to a computation module for performing the steps. The steps includes: obtaining a coach window from the coach exercise data, obtaining a user window from the user exercise data, in which a length of the user window is longer than that of the coach window; searching a user segment from the user window that is most similar to the coach window; for a first exercise type, calculating an exercise score according to stabilities of the user segment and the coach window; and for a second exercise type, calculating the exercise score based on a difference between the user segment and the coach window by a deduction mechanism.

**BRIEF DESCRIPTION OF THE DRAWINGS**

The disclosure can be more fully understood by reading the following detailed description of the embodiment, with reference made to the accompanying drawings as follows.

FIG. 1A is a schematic diagram illustrating a scoring system in accordance with an embodiment.

FIG. 1B is a flow chart for the exercise system in accordance with an embodiment.

FIG. 2 is a schematic diagram illustrating coach exercise data and user exercise data in accordance with an embodiment.

FIG. 3 is a flow chart of a scoring method in accordance with an embodiment.

**DETAILED DESCRIPTION**

The usage of "first", "second", "third", etc. in the specification should be understood for identifying units or data described by the same terminology, but are not referred to a particular order or sequence.

A combination of a smart device and a cloud application service is provided in a scoring method for an exercise course. When a user exercises according to an exercise course video, movement data of several body portions of the user is acquired. The movement data of the user is compared with movement data of a coach with respect to the body portions so as to assess the exercise result of the user. Accordingly, the user can know whether he is exercising correctly to improve the exercise effect and the willingness to exercise. In particular, different approaches will be used to calculate the score for different exercise types, and for some exercise types or fast actions that are not important in gesture, a greater tolerance will be given.

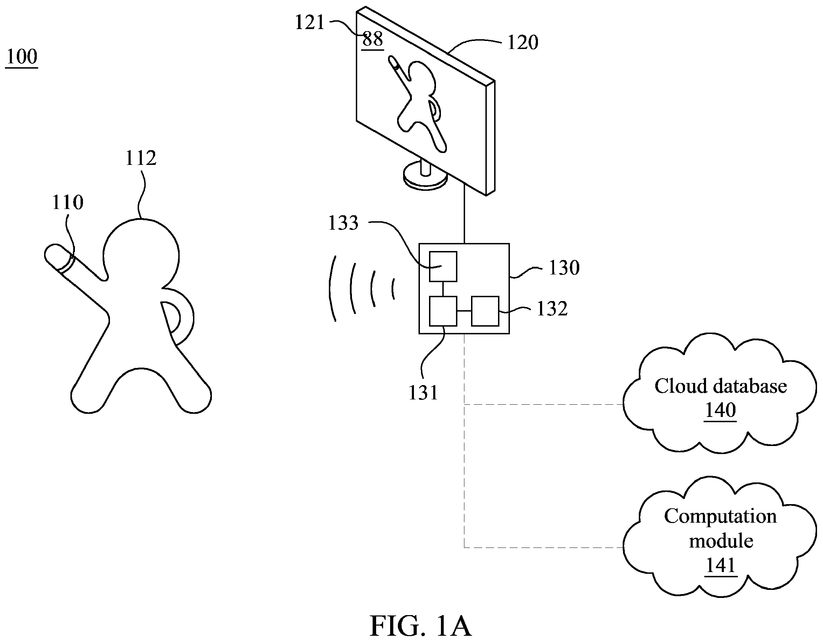

FIG. 1A is a schematic diagram illustrating a scoring system in accordance with an embodiment. Referring to FIG. 1A, a scoring system 100 includes at least one wearable device 110, a display 120, and a smart device 130.

The wearable device 110 is, for example, a sports bracelet worn by a user 112. However, the wearable device 110 may also be a watch, a belt, or any device that can be worn by the user. The wearable device 110 includes an inertial measurement unit (IMU) which at least includes a tri-axial gravity sensor for sensing an X-axis acceleration value, a Y-axis acceleration value, and a Z-axis acceleration value. In some embodiments, the IMU further includes an angular velocity sensor and/or a magnetometer. The data measured by the IMU is referred to as user exercise data. The wearable device 110 may also include a wireless communication module such as Bluetooth module, a wireless fidelity (Wi-Fi) module or any other suitable low power wireless communication module. In some embodiments, the wearable device 110 also includes a display panel and some other units that are not limited in the disclosure. The display 120 displays a course video in which a coach is demonstrating actions.

The smart device 130 includes a processor 131, a memory 132, and a wireless communication module 133. The memory 132 stores instructions which are executed by the processor 131. The processor 131 is, for example, a central processing unit, a microprocessor, a microcontroller, a digital signal processor, an image processing chip, an application-specific integrated circuit which is not limited in the disclosure. The wireless communication module 133 is, for example, a Bluetooth module, a Wi-Fi module or any other suitable low power wireless communication module for communicatively connecting to the wearable device 110 and receiving the user exercise data from the wearable device 110.

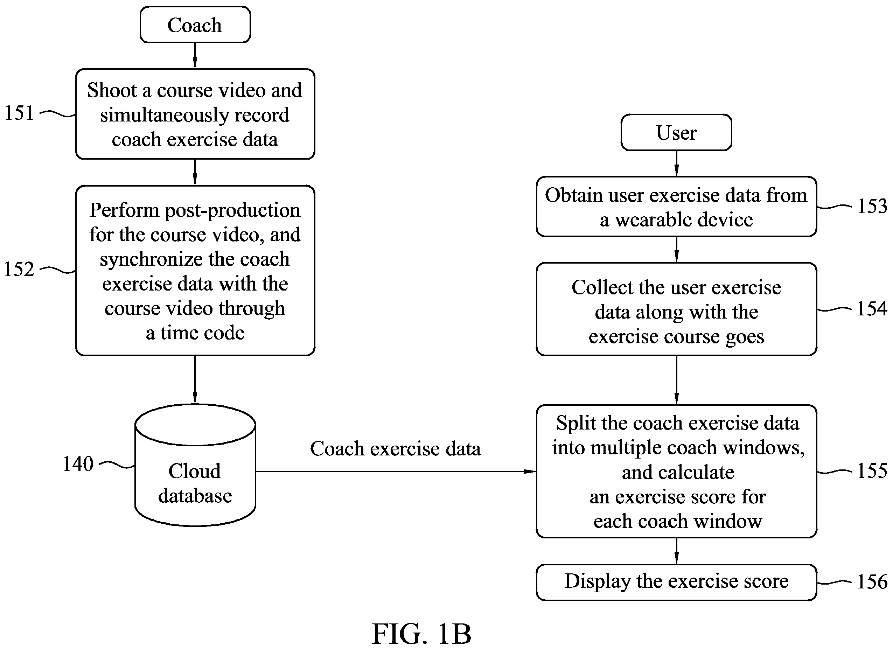

FIG. 1B is a flow chart for the scoring system 100 in accordance with an embodiment. Referring to FIG. 1A and FIG. 1B, a course video is shot and coach exercise data is simultaneously recorded in step 151. In some embodiments, a coach is shot by several cameras which are disposed at different locations with different shooting angles to generate several films that are used to generate the course video. In particular, during the shooting, the coach wears one or multiple wearable devices 110 where the data sensed by the IMU is recorded as the coach exercise data. For example, the coach exercise data includes multiple sample points, and each sample point includes an X-axis acceleration value, a Y-axis acceleration value, and a Z-axis acceleration value.

In step 152, post-production for the course video is performed, in which the coach exercise data is synchronized with the course video through a time code such as Society of Motion Picture and Television Engineers (SMPTE), but any other format of time code may be adopted in another embodiment which is not limited in the disclosure. Accordingly, it can be known which sample point of the coach exercise data corresponds to which clip or frame of the course video through the time code. For example, if a sample frequency of the IMU of the wearable device 110 is for example but not limited to 25Hz, then the coach exercise data corresponding to a minute of video will have 60 x 25 = 1500 sample points. Next, the course video and the coach exercise data are stored in a cloud database 140.

When a user is about to exercise, the smart device 130 obtains the course video and the coach exercise data form the cloud database 140, and plays the course video through the display 120. Meanwhile, in step 153, the smart device 130 obtains user exercise data from the wearable device 110 worn by the user. In some embodiments, the smart device 130 obtains the user exercise data from the wearable device 110 before the course video is played, but some of these data will be discarded until the course video is played, and then the remaining data are stored as the user exercise data. As a result, the obtained user exercise data completely corresponds to the course video. It can be known which sample point of the user exercise data corresponds to which frame of the course video.

In step 154, the user exercise data is collected along with the exercise course goes. Since the corresponding relationship between the user exercise data and the course video is obtained in the step 153 and the corresponding relationship between the course video and the coach exercise data is obtained by the SMPTE time code, the corresponding user exercise data and coach exercise data can be obtained according to a frame number of the course video. In other words, it can be known which sample point of the user exercise data corresponds to which sample point of the coach exercise data according to the frame number.

In step 155, the coach exercise data is split into multiple coach windows, and an exercise score 121 is calculated for each coach window. In step 156, the exercise score 121 is displayed, and consequently the user 112 can be aware whether he or she is doing the movement correctly, resulting in improving the exercise effect and willingness to exercise. The exercise scores are calculated by the processor 131 in some embodiments, but the exercise scores may be calculated by a server on the cloud or any other electric device in other embodiments. For example, in FIG. 1A, the smart device 130 may be communicatively connected to a computation module 141 such as a server, a virtual machine or a web application providing a computing service, which is not limited in the disclosure. The processor 131 can transmit the coach exercise data and the user exercise data to the computation module 141, and after the exercise scores are calculated by the computation module 141, the scores are transmitted back to the processor 131. In some embodiments, the exercise scores are calculated while the user exercise data is received. In other words, the exercise scores may be calculated and displayed in real time. In some embodiments, the exercise scores are calculated when the exercise course stops or ends. In some embodiments, when the quantity of the user exercise data is enough, the exercise scores are calculated by the smart device 130. In some embodiments, the user exercise data is transmitted to the computation module 141 after the exercise stops or ends. The calculation of the exercise score will be described in detail below.

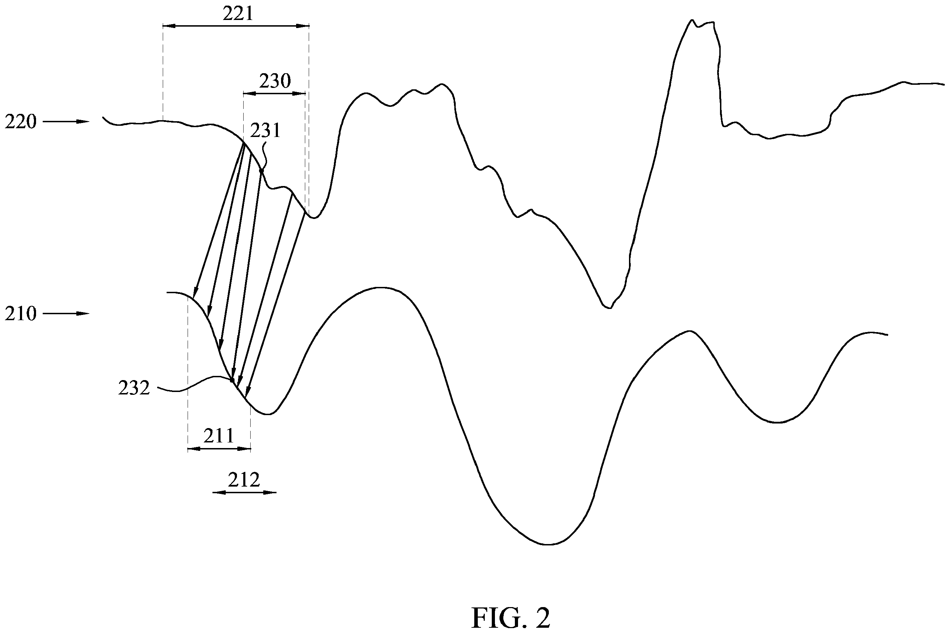

FIG. 2 is a schematic diagram illustrating coach exercise data and user exercise data in accordance with an embodiment. Coach exercise data 210 and user exercise data 220 are illustrated as one-dimensional signals in FIG. 2 for description. In practical, each sample point should have multiple acceleration values (i.e. a vector), and the coach exercise data 210 and the user exercise data 220 are sets of vectors. In detail, the coach exercise data 210 is represented

as $F_c[i] = [a_{i,x}^c, a_{i,y}^c, a_{i,z}^c]$ where i is a positive integer indicating ith sample point. $a_{i,x}^c, a_{i,y}^c,$ and $a_{i,z}^c$ represent the X acceleration value, Y acceleration value, and Z acceleration value respectively at the ith sample point of the coach exercise data 210. The user exercise data 220 is represented as $F_s[i] = [a_{i,x}^s, a_{i,y}^s, a_{i,z}^s]$ where $a_{i,x}^s, a_{i,y}^s,$ and $a_{i,z}^s$ represent the X acceleration value, the Y acceleration value, and the Z acceleration value respectively at the ith sample point of the user exercise data 220. The coach exercise data 210 and the user exercise data 220 correspond to the same course video, and thus the score should be high if the coach exercise data 210 is similar to the user exercise data 220. Note that the length of the coach exercise data 210 may be not identical to that of the user exercise data 220. In some embodiments, if the sample frequency of the user exercise data 220 is different from that of the coach exercise data 210, the data having greater sample frequency may be resampled so that the sample frequencies are the same.

In some embodiments, if the IMU further includes the angular velocity sensor, an algorithm for fusing six-axis data will be applied to obtain the vectors $F_c[i]$ and $F_s[i]$. The fusion data can be used to properly distinguish the gesture of the action, determine more various exercise types, and provide a better discrimination rate. If the IMU further includes a three-axis magnetometer, an algorithm for fusing nine-axis data may be applied to obtain the vectors $F_c[i]$ and $F_s[i]$. The algorithm may be an attitude and heading reference system (AHRS) algorithm which is not limited in the disclosure.

A coach window 211 is obtained from the coach exercise data 210. The length of the coach window 211 may be in a range from 2 seconds to 10 seconds that is not limited in the disclosure. In some embodiments, the length of the coach window 211 is determined based on an exercise type. On the other hand, a user window 221 is obtained from the user exercise data 220. The length of the user window 221 is longer than that of the coach window 211. For example, the beginning point of the user window 221 may precede the beginning point of the coach window 211, and the end point of the user window 221 follows the end point of the coach window 211 because user's actions may be too late or too early when he imitates the coach, and longer length of the user window 221 can provide greater tolerance. If the length of the coach window 211 is 4 seconds and the sample frequency is 25 Hz, then the coach window 211 will have $M = 4 \times 25 = 100$ sample points. If the beginning point of the user window 221 is one second earlier than that of the coach window 211, the end point of the user window 221 is one second later than that of the coach window 211, and the sample frequency of the user window 221 is also 25 Hz, then the user window 221 will have total of $N = 100 + 2 \times 25 = 150$ sample points.

A user segment 230 of the user window 221 that is most similar to the coach window 211 is searched. In some embodiments, an open-begin-end dynamic time warping (OBE-DTW) algorithm is performed to the user window 221 and the coach window 211 to obtain a user segment 230. People in the art should be able to understand the OBE-DTW algorithm which will be not described in detail herein. The OBE-DTW algorithm releases the constraints of identical beginning points and identical end points so as to find a partially matched segment in the user window 221. The algorithm can be represented as the following equations (1) to (3). The coach window 211 is written as $F_c$ in the following equations for simplification.

$$D_{OBE}(F_c, F_s) = \min_{1 \leq d \leq e \leq N} D\left(F_c, F_s^{(d,e)}\right) \quad (1)$$

$$F_s^{(d,e)} = (F_s[d], F_s[d+1], \ldots, F_s[e]]) \quad (2)$$

$$D\left(F_c, F_s^{(d,e)}\right) = Cost_{OBE-DTW} \times \left(1 + \frac{|N_{matcted} - M| \times a}{M}\right) \quad (3)$$

$F_s^{(d,e)}$ is a subset (i.e. segment) of the user window 221 that includes the $d^{th}$ sample point to the $e^{th}$ sample point where d and e are positive integers. The length of the segment $F_s^{(d,e)}$ may be identical or shorter from that of the user window 221 since $1 \leq d \leq e \leq N$. In other words, the $d^{th}$ sample point to the $e^{th}$ sample of the user window 221 constitute the user segment 230 which is matched to the sample points of the coach window $F_c$. The algorithm is to find the positive integers $d$ and $e$ so that the difference $D\left(F_c, F_s^{(d,e)}\right)$ is minimized. $Cost_{OBE\text{-}DTW}$ is the average of the errors between the matched sample points determined by the OBE-DTW algorithm. For example, a sample point 231 of the user segment 230 is matched to a sample point 232 of the coach window 211 by the OBE-DTW algorithm. Note that one sample point of the user segment 230 may be matched to multiple sample points of the coach window 211. In addition, the matched sample points of the user window 221 may not be continuous (i.e. some sample points of the user window 221 are not matched to the coach window 211). Different measurement of the difference may be adopted for different exercise types. If the exercise type is mainly related to postures, then $Cost_{OBE\text{-}DTW}$ may be the average of cosine similarities of the matched sample points. If the exercise type is mainly related to forces, then $Cost_{OBE\text{-}DTW}$ may be the average of L2 norm (i.e. Euclidean distance) between the matched sample points. Moreover, $N_{matcted}$ is the length of the found user segment 230 and a is a real number such as 2. The equation (3) is different from the conventional OBE-DTW to adjust the difference $D\left(F_c, F_s^{(d,e)}\right)$ based on the length of the user segment 230. The user segment 230 having the length close to the length of the coach window 221 is preferred. As shown in FIG. 2, the user's movement is slightly behind the coach's movement, and thus a portion at the beginning of the user window 221 is discarded based on the disclosed algorithm. Accordingly, more tolerance is provided to the user's delayed movements.

After the user segment 230 is found, an exercise type is determined to adopt the corresponding approach to calculate an exercise score. At least two exercise types are set herein. The first exercise type is related to stable movement, and the second exercise type is related to general or fast movement.

In some embodiments, the absolutes of the acceleration values of the X, Y and Z axes are summed up to obtain a force $x[i]$ at the $i^{th}$ sample point which is written as the following equation (4).

$$x[i] = |a_{i,x}| + |a_{i,y}| + |a_{i,z}| \qquad (4)$$

The acceleration values $a_{i,x}$, $a_{i,y}$, and $a_{i,z}$ may belong to the coach exercise data or the user exercise data. The forces of the coach exercise data are adopted when determining the exercise type. Next, the average, stand deviation, or mean absolute deviation of all forces $x[i]$ is calculated to classify the exercise type. For example, when the mean absolute deviation is less than a threshold, it is determined to be the first exercise type, or otherwise the second exercise type. In some embodiments, the exercise type is determined based on the sports of the coach exercise data such as boxing, Latin dance, interval strength training, and yoga. In some embodiments, the exercise type is determined based on the length of the coach window 211 or the user window 221. In some embodiments, a weight (e.g. in a range from 0 to 1) of the first exercise type and a weight (e.g. in a range from 0 to 1) of the second exercise type are calculated for the user window 221. Two scores corresponding to the first and second exercise types are calculated and summed based on the weights. In some embodiments, different user windows may have different exercise types or different weights. The classification of the exercise type is not limited in the disclosure. The calculation of the exercise score with respect to each exercise type is described as follows.

[First exercise type]

The aforementioned mean absolute deviation may be represented as the following equation (5). The mean absolute (MAD) is also referred to as stability.

$$MAD = \frac{1}{N} \sum_{i=1}^{N} |x[i] - \mu| \qquad (5)$$

$\mu$ is the mean of all forces $x[i]$. The equation (5) may be used to calculate the stability of the user exercise data that is referred to as user stability $MAD_{user}$ herein. The equation (5) may also be used to calculate the stability of the coach exercise data while the positive integer N is replaced with M, and the result is referred to as coach stability $MAD_{ref}$. The exercise score is calculated according to the user stability $MAD_{user}$ and the coach stability $MAD_{ref}$. The closer the two stabilities are, the higher the exercise score is. In some embodiments, a stability ratio r of the user stability $MAD_{user}$ to the coach stability $MAD_{ref}$ is calculated as the following equation (6).

$$r = \frac{MAD_{user}}{MAD_{ref}}, r \in (0, \infty) \qquad (6)$$

Then, the stability ratio is shifted, resized, and flipped based on a logit function so that the stability ratio is transformed into a stability score as the following equations (7)-(9).

$$S_{stability}(r) = 100 \times \begin{cases} S_{base} + (1 - S_{base}) \times f(\frac{1}{r}), if\ r < 1 \\ S_{base} - S_{base} \times f(r), otherwise \end{cases} \qquad (7)$$

$$f(x) = \frac{1}{q} ln(\frac{1-T(x)}{T(x)}) \qquad (8)$$

$$T(x) = \frac{x-1}{2(r_{bound}-1)} + 0.5 \qquad (9)$$

$S_{stability}(r)$ is the stability score. $S_{base}$ is a real number for indicating the score when $r=1$. $r_{bound}$ is a real number for defining a boundary of the stability ratio r. In the example of $r_{bound}$ = 5, a score of 100 is obtained when the user stability is one fifth of the coach stability (i.e. $r$ =1/5) and a score of zero is obtained when the user stability is five times of the coach stability (i.e. $r$=5). q is a real number for controlling the trend of the equation (8). In some embodiments, the logit function is implemented as a lookup table.

In response to the diversity of exercise situations, if the scoring conditions are too strict, it will cause many actions that are seen as static to be classified as dynamic actions, and will also cause too many false judgments. In order to solve this problem, a double threshold method is used to divide static motion into true static and fuzzy static. To be specific, an upper bound threshold $t_h$ and a true static threshold t are set herein, and the former is greater than the latter. These two thresholds define a first range [0,t] and a second range (t, $t_h$). The coach exercise data is divided into multiple windows, and the coach stability $MAD_{ref}$ is calculated for each window. Times of the coach stabilities $MAD_{ref}$ falling within the first range [0,t] are counted as a first number of times. Times of the coach stabilities $MAD_{ref}$ falling within the second range (t, $t_h$) are counted as a second number of times. The first number of times is divided by a sum of the first number of times and the second number of times to obtain a stability weight w as the following equation (10).

$$w = \frac{count_{under}}{count_{under} + count_{fuzzy}} \qquad (10)$$

$count_{under}$ is the first number of times. $count_{fuzzy}$ is the second number of times. Last, the exercise score is calculated according to the stability score $S_{stability}(r)$ and the stability weight w as the following equation (11).

$$Score = S_{stability} \times w + (1 - w) \times S_1 \qquad (11)$$

Score is the exercise score. $S_1$ is a real number such as 90.

[Second exercise type]

The exercise score is calculated based on a difference between the user segment and the coach window by a deduction mechanism for the second exercise type. First, a force difference between the user segment and the coach window is calculated. The force difference is shifted and resized based on a sigmoid function. To be specific, the sigmoid function is represented as the following equation (12).

$$\sigma_{(m,q)}(x) = \frac{1}{1 + e^{-q(x-m)}} \qquad (12)$$

*m* and *q* are parameters. In some embodiments, the sigmoid function is implemented as a lookup table. The force of each sample point of the coach window is represented as the following equation (13).

$$mag_c[i] = \|F_c[i]\|, i = 1,2,...M \qquad (13)$$

||·|| is L2 norm. M is the length of the coach window. A dynamic factor for the coach exercise data is calculated as the following equation (14).

$$mag_{upper}[i] = std(mag_c, i, w) + mean(mag_c, i, w),$$

$$i = 1,2,...,length(mag_c) \qquad (14)$$

w is the size of the window. *std(mag_c,i,w)* is the stand deviation of a set from the $i^{th}$ sample point to the $(i+w)^{th}$ sample point of the force *mag_c[]*. *mean(mag_c,i,w)* is the mean of the set from the $i^{th}$ sample point to the $(i+w)^{th}$ sample point of the force *mag_c[]*. The transformation of the sigmoid function is written as the following equation (15).

$$D_m = \frac{100}{M} \sum_{(i_c,i_s)\epsilon P} \sigma_{m_m,q_m}\left(\frac{|mag_c[i_c]-\|F_s[i_s]\||}{mag_{upper}[i_c]}\right) \qquad (15)$$

*P* is a set including the matched sample point pairs between the user segment and the coach window that are determined by the OBE-DTW algorithm. For example, the sample point 231 and the sample point 232 of FIG. 2 constitute a sample point pair. $(i_c,i_s)$ is a sample point pair in the set *P*. $m_m$, $q_m$ are real numbers for controlling the tolerance of the force difference. $D_m$ is the force difference and also is the score to be deducted. The dynamic factor $mag_{upper}[i_c]$ is used to adjust the force difference $D_m$ adaptively based on the coach exercise data. When the stand deviation of the forces of the coach exercise data is great, it means the movements are hard to imitate, and therefore the score $D_m$ to be deducted is less. From another aspect, the force difference $D_m$ is calculated based on the difference between the L2 norms $\|F_s[i_s]\|$ of the user segment and the L2 norms $\|F_c[i]\|$ of the coach window at matched sample points.

In addition, an angular difference is calculated according to the following equation (16).

$$D_a = \frac{100}{M} \sum_{(i_c,i_s)\epsilon P} \sigma_{m_a,q_a}\left(cos^{-1}\frac{F_c[i_c]\cdot F_s[i_s]}{\|F_c[i_c]\|\cdot\|F_s[i_s]\|}\right) \qquad (16)$$

$m_a$ and $q_a$ are real numbers for controlling the tolerance of the angular difference. $D_a$ is the angular difference and also is the score to be deducted. From another aspect, the angular difference $D_a$ is based on the cosine similarities between the user segment and the coach window at matched sample points. The force difference $D_m$ is used to determine if the force of the movement is correct, and the angular difference $D_a$ is used to determine if the orientation of the movement is correct. When the user follows the coach correctly, the force and angular differences are small, and thus the score to be deducted is small. When the movement of the user significantly differs from that of the coach, one of the force and angular difference may still be small, and a high score should not be given in this case. Therefore, two weights are set herein to mix the force difference $D_m$ and the angular difference $D_a$.

To be specific, a force weight $w_m$ and an angular weight $W_a$ are set in the embodiment. When a larger one of the force difference $W_m$ and the angular difference $W_a$ is greater than an error threshold (i.e. indicating that the movement difference is great), one of the weights that corresponds to the larger difference is increased, and the other weight is decreased. Accordingly, a high score will not be given. In some embodiments, the step of increasing the weight is based on the sigmoid function as the following equation (17).

$$DAM_{q,m}(w,d) = 1 - \frac{w}{1+e^{-q(1-d)-m}} \qquad (17)$$

The sum of the force weight $W_m$ and the angular weight $W_a$ is equal to 1. The force weight $W_m$ and the angular weight $W_a$ may be determined according to the following pseudo codes.

$$\text{If } D_a > D_m:$$

$$w_a := DAM_{q,m}(w_a, D_a)$$

$$w_m := 1 - w_a$$

$$\text{else}$$

$$w_m := DAM_{q,m}(w_m, D_m)$$

$$w_a := 1 - w_m$$

In the equation (17), $q$ is a real number for deciding the increasing magnitude, and $m$ is a real number for deciding the shift of the sigmoid function. The real number $m$ is also referred to the said error threshold. For example, when the angular difference $D_a$ is the larger difference, the angular difference $D_a$ will be inputted to the sigmoid function. The angular weight $W_a$ will increase quickly if the angular difference $D_a$ is greater than the error threshold $m$ or otherwise the angular weight $W_a$ does not change a lot.

Last, the exercise is calculated according to the force difference, the angular difference, the force weight, and the angular weight as the following equation (18).

$$Score = 100 - (D_a \times w_a + D_m \times w_m) \qquad (18)$$

Referring to FIG. 2, after the exercise score for the coach window 211 is calculated, an exercise score for the next coach window 212 is calculated. In particular, the coach window 212 and the coach window 211 partially overlap. For example, the coach window 211 may slide to the right for a small distance to get the coach window 212. A too small sliding distance will produce many unnecessary calculations and cannot improve the resolution of motion analysis. An excessively large sliding distance will reduce calculations but also reduce the resolution of motion analysis. In some embodiments, the sliding distance is 0.15 seconds.

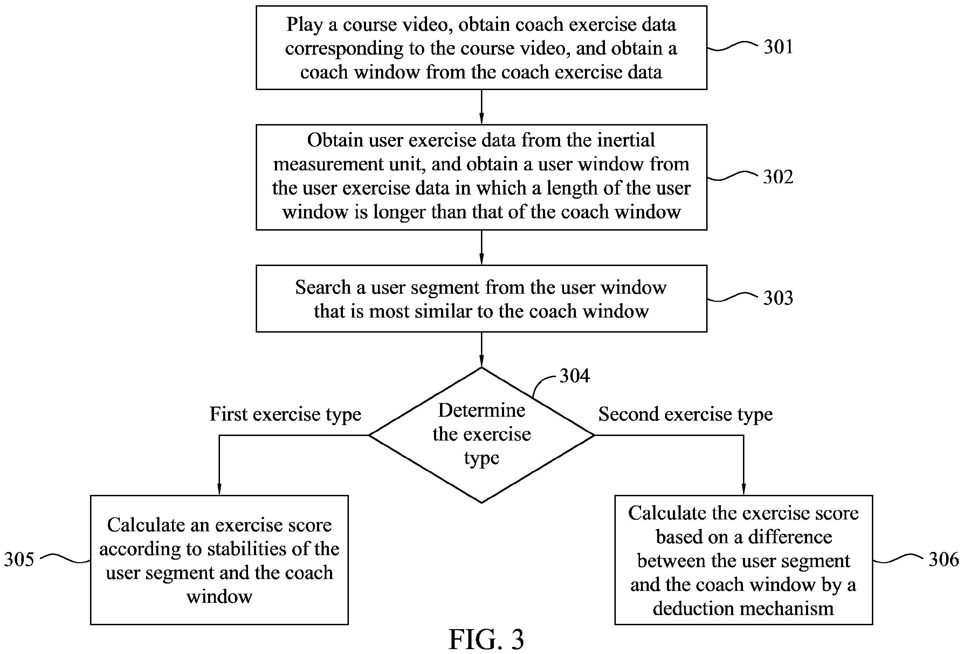

FIG. 3 is a flow chart of a scoring method in accordance with an embodiment. Referring to FIG. 3, in step 301, a course video is played, coach exercise data corresponding to the course video is obtained, and a coach window is obtained from the coach exercise data. In step 302, user exercise data is obtained from an inertial measurement unit, and a user window is obtained from the user exercise data in which a length of the user window is longer than that of the coach window. In step 303, a user segment is searched from the user window that is most similar to the coach window. In step 304, an exercise type is determined. If it is the first exercise type, an exercise score is calculated according to stabilities of the user segment and the coach window in step 305. If it is the second exercise type, the exercise score is calculated based on a difference between the user segment and the coach window by a deduction mechanism in step 306. In some embodiments, the step 304 may be replaced with calculating the weight of the first exercise type and the weight of the second exercise type, and both of the steps 305 and 306 are executed. The exercise scores calculated by the steps 305 and 306 will be summed up based on the two weights. As mentioned above, the two weights may be determined according to the sports such as boxing, Latin dances, interval strength training, yoga by a lookup table. Alternatively, the two weights may be determined based on the length of the coach window 211 (or the user window 221). However, all the steps in FIG. 3 have been described in detail above, and therefore the description will not be repeated. Note that the steps in FIG. 3 can be implemented as program codes or circuits, and the disclosure is not limited thereto. In addition, the method in FIG. 3 can be performed with the aforementioned embodiments, or can be performed independently. In other words, other steps may be inserted between the steps of the FIG. 3.

In some embodiments, the aforementioned L2 norm may be replaced with L1 norm, max norm, histogram distance, or any suitable distance. In some embodiments, the OBE-DTW may be replaced with other types of DTW or similarity algorithms such as longest common subsequence (LCS).

**Claims**

1. A scoring method for a system comprising a wearable device (110) which comprises an inertial measurement unit, wherein the scoring method is **characterized by** comprising:

   playing a course video, obtaining coach exercise data (210) corresponding to the course video, and obtaining a coach window (211) from the coach exercise data;
   obtaining user exercise data (220) from the inertial measurement unit and obtaining a user window (221) from the user exercise data (220), wherein a length of the user window (221) is longer than that of the coach window (211);
   searching a user segment (230) from the user window that is most similar to the coach window (211);
   for a first exercise type, calculating an exercise score according to stabilities of the user segment (230) and the coach window (211); and
   for a second exercise type, calculating the exercise score based on a difference between the user segment (230) and the coach window (211) by a deduction mechanism.

2. The scoring method of claim 1, wherein a beginning point of the user window (221) precedes a beginning point of the coach window (211), an end point of the user window (221) follows an end point of the coach window (211), and the step of searching the user segment (230) from the user window (221) that is most similar to the coach window (211) comprises:
   performing an open-begin-end dynamic time warping algorithm to the user window (221) and the coach window (211) to obtain the user segment (230).

3. The scoring method of claim 1, wherein the step of calculating the exercise score according to the stabilities of the user segment and the coach window comprises:

   calculating a mean absolute deviation of forces at a plurality of sample points of the user segment (230) as a user stability;
   calculating a mean absolute deviation of forces at a plurality of sample points of the coach window (211) as a coach stability; and
   calculating a stability ratio between the user stability and the coach stability.

4. The scoring method of claim 3, wherein the step of calculating the exercise score according to the stabilities of the user segment and the coach window further comprises:

   transforming the stability ratio into a stability score based on a logit function;
   calculating the corresponding coach stability for each window of the coach exercise data, counting times of the coach stabilities falling within a first range as a first number of times, and counting times of the coach stabilities falling within a second range as a second number of times;
   dividing the first number of times by a sum of the first number of times and the second number of times to obtain a stability weight; and
   calculating the exercise score according to the stability score and the stability weight.

5. The scoring method of claim 4, wherein the step of calculating the exercise score according to the stability score and the stability weight is based on an equation (1):

$$Score = S_{stability} \times w + (1 - w) \times S_1 \qquad (1)$$

   wherein *Score* is the exercise score, $S_{stability}$ is the stability score, w is the stability weight, and $S_1$ is a real number.

6. The scoring method of claim 1, wherein the step of calculating the exercise score based on the difference between the user segment (230) and the coach window (211) by a deduction mechanism comprises:

   calculating a force difference and an angular difference between the user segment (230) and the coach window (211);
   setting a force weight and an angular weight, and when larger one of the force difference and the angular difference is greater than an error threshold, increasing one of the force weight and the angular weight that corresponds to the larger one of the force difference and the angular difference; and

calculating the exercise score based on the force difference, the angular difference, the force weight, and the angular weight.

7. The scoring method of claim 6, wherein the force difference is based on differences between L2 norms of the user segment and L2 norms of the coach window at matched sample points,

wherein the angular difference is based on cosine similarities between the user segment (230) and the coach window (211) at the matched sample points.

8. The scoring method of claim 7, wherein the step of calculating the exercise score based on the force difference, the angular difference, the force weight, and the angular weight is based on an equation (1):

$$Score = 100 - (D_a \times w_a + D_m \times w_m) \qquad (1)$$

wherein *Score* is the exercise score, $D_a$ is the angular difference, $W_a$ is the angular weight, $D_m$ is the force difference, and $W_m$ is the force weight.

9. A scoring system (100) comprising:

a wearable device (110) comprising an inertial measurement unit configured to obtain user exercise data (220);

wherein the scoring system is **characterized by** further comprising a smart device (130) communicatively connected to the wearable device (110) for receiving the user exercise data (220) and configured to play a course video through a display (120),

wherein the smart device (130) is configured to perform a plurality of steps or transmit the user exercise data (220) and coach exercise data (210) corresponding to the coach video to a computation module (141) for performing the steps comprising:

obtaining a coach window (211) from coach exercise data (210), obtaining a user window (221) from the user exercise data (220), wherein a length of the user window (220) is longer than that of the coach window (211);

searching a user segment (230) from the user window (221) that is most similar to the coach window (211);

for a first exercise type, calculating an exercise score according to stabilities of the user segment (230) and the coach window (211); and

for a second exercise type, calculating the exercise score based on a difference between the user segment (230) and the coach window (211) by a deduction mechanism.

FIG. 1A

EP 3 886 109 A1

```
         ┌──────────┐
         │  Coach   │
         └──────────┘
              │
              ▼
    ┌─────────────────────┐
    │ Shoot a course video│
151 │ and simultaneously  │
    │ record coach        │
    │ exercise data       │
    └─────────────────────┘
              │
              ▼
    ┌─────────────────────┐
    │ Perform post-       │
    │ production for the  │
    │ course video, and   │
152 │ synchronize the     │
    │ coach exercise data │
    │ with the course     │
    │ video through       │
    │ a time code         │
    └─────────────────────┘
              │
              ▼
```

```
         ┌──────────┐
         │   User   │
         └──────────┘
              │
              ▼
    ┌─────────────────────┐
    │ Obtain user exercise│  153
    │ data from a         │
    │ wearable device     │
    └─────────────────────┘
              │
              ▼
    ┌─────────────────────┐
    │ Collect the user    │
    │ exercise data along │  154
    │ with the exercise   │
    │ course goes         │
    └─────────────────────┘
              │
              ▼
```

140 Cloud database  → Coach exercise data →

```
    ┌─────────────────────┐
    │ Split the coach     │
    │ exercise data into  │
    │ multiple coach      │
    │ windows, and        │  155
    │ calculate an        │
    │ exercise score for  │
    │ each coach window   │
    └─────────────────────┘
              │
              ▼
    ┌─────────────────────┐
    │ Display the         │  156
    │ exercise score      │
    └─────────────────────┘
```

FIG. 1B

FIG. 2

EP 3 886 109 A1

```
┌─────────────────────────────────────┐
│ Play a course video, obtain coach     │
│ exercise data corresponding to the   │──── 301
│ course video, and obtain a coach      │
│ window from the coach exercise data   │
└─────────────────────────────────────┘
                  │
                  ▼
┌─────────────────────────────────────┐
│ Obtain user exercise data from the    │
│ inertial measurement unit, and obtain │
│ a user window from the user exercise  │──── 302
│ data in which a length of the user    │
│ window is longer than that of the     │
│ coach window                          │
└─────────────────────────────────────┘
                  │
                  ▼
┌─────────────────────────────────────┐
│ Search a user segment from the user   │──── 303
│ window that is most similar to the    │
│ coach window                          │
└─────────────────────────────────────┘
                  │
                  ▼
              ╱────────╲  ── 304
First exercise type  ╱ Determine ╲  Second exercise type
          ╱   the exercise   ╲
          ╲      type       ╱
              ╲────────╱
    │                           │
    ▼                           ▼
┌──────────────────┐    ┌──────────────────┐
│ Calculate an      │    │ Calculate the     │
│ exercise score    │    │ exercise score    │
305 ─│ according to      │    │ based on a        │── 306
│ stabilities of the│    │ difference        │
│ user segment and  │    │ between the user  │
│ the coach window  │    │ segment and the   │
└──────────────────┘    │ coach window by a │
                        │ deduction         │
                        │ mechanism         │
                        └──────────────────┘
```

FIG. 3

EP 3 886 109 A1

Europäisches Patentamt
European Patent Office
Office européen des brevets

**EUROPEAN SEARCH REPORT**

Application Number

EP 20 18 5591

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/247716 A1 (CHUANG LUNG-FEI [TW]) 15 August 2019 (2019-08-15) * paragraphs [0005] - [0017], [0046] - [0055]; claims; figures * ----- | 1-9 | INV. G16H20/30 A63B71/06 |
| Y | WO 2019/153454 A1 (CHUANG LUNG FEI [CN]) 15 August 2019 (2019-08-15) * claims; figures * ----- | 1-9 | |

TECHNICAL FIELDS
SEARCHED (IPC)

G16H
A63B

The present search report has been drawn up for all claims

| Place of search | Date of completion of the search | Examiner |
|---|---|---|
| Munich | 7 December 2020 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

16

**ANNEX TO THE EUROPEAN SEARCH REPORT
ON EUROPEAN PATENT APPLICATION NO.**

EP 20 18 5591

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2020

| Patent document cited in search report | | Publication date | Patent family member(s) | | Publication date |
|---|---|---|---|---|---|
| US 2019247716 | A1 | 15-08-2019 | JP<br>TW<br>US | 2019136493 A<br>201934175 A<br>2019247716 A1 | 22-08-2019<br>01-09-2019<br>15-08-2019 |
| WO 2019153454 | A1 | 15-08-2019 | CN<br>EP<br>WO | 110148072 A<br>3753610 A1<br>2019153454 A1 | 20-08-2019<br>23-12-2020<br>15-08-2019 |

EPO FORM P0459

For more details about this annex : see Official Journal of the European Patent Office, No. 12/82